(19) Europäisches Patentamt
European Patent Office
Office européen des brevets

(11) EP 1 559 394 B1

(12) FASCICULE DE BREVET EUROPEEN

(45) Date de publication et mention de la délivrance du brevet:
**31.03.2010 Bulletin 2010/13**

(21) Numéro de dépôt: **05290187.3**

(22) Date de dépôt: **27.01.2005**

(51) Int Cl.:
***A61K 8/19*** *(2006.01)* ***A61K 8/92*** *(2006.01)*
***A61K 8/891*** *(2006.01)* ***A61K 8/81*** *(2006.01)*
***A61Q 1/00*** *(2006.01)* ***A61Q 1/02*** *(2006.01)*
***A61Q 1/12*** *(2006.01)*

# (54) Composition cosmétique de type poudre compacte à phase grasse solide

Kosmetische Kompaktpuderzusammensetzung enthaltend eine feste fette Phase

Pressed powder cosmetic composition containing a solid fatty phase

(84) Etats contractants désignés:
**AT BE BG CH CY CZ DE DK EE ES FI FR GB GR HU IE IS IT LI LT LU MC NL PL PT RO SE SI SK TR**

(30) Priorité: **27.01.2004 FR 0450148**

(43) Date de publication de la demande:
**03.08.2005 Bulletin 2005/31**

(73) Titulaire: **L'Oréal**
**75008 Paris (FR)**

(72) Inventeurs:
- **Martin, Guenaelle**
**78360 Montesson (FR)**
- **Themens, Agnès**
**92340 Bourg la Reine (FR)**
- **Collineau, Maïtena**
**75005 Paris (FR)**

(74) Mandataire: **Le Coupanec, Pascale A.M.P.**
**Nony & Associés**
**3 rue de Penthièvre**
**75008 Paris (FR)**

(56) Documents cités:
**EP-A- 0 132 631 EP-A- 0 605 284**
**EP-A- 0 657 486 EP-A- 0 717 979**
**EP-A- 1 159 950 EP-A- 1 277 458**
**FR-A- 2 798 287 FR-A- 2 848 820**
**FR-A- 2 851 462 FR-A- 2 854 324**

- **DATABASE CAPLUS [Online] XP002294971 extrait de STN Database accession no. 1997:2936 & JP 08 259429 A (SHISEIDO CO.) 8 octobre 1996 (1996-10-08)**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne une composition cosmétique pour le maquillage et/ou le soin de la peau, notamment du visage.

Plus particulièrement, les compositions considérées selon l'invention peuvent constituer un produit de maquillage, par exemple du visage, ayant notamment des propriétés de soin et/ou de traitement non thérapeutique. Il peut s'agir de compositions cosmétiques dites matifiantes, c'est-à-dire destinées à réduire la brillance des peaux grasses et/ou améliorer la tenue du maquillage à long terme, c'est-à-dire prévenir la dégradation visuelle au cours de la journée.

De nombreuses compositions de maquillage de ce type se présentent sous forme de poudre compacte comprenant généralement une phase grasse, appelée classiquement « liant », et une phase pulvérulente comprenant notamment des pigments et/ou des charges. Le liant a pour fonction principale de garantir une cohésion suffisante de la composition finale, notamment de manière a lui éviter une fragmentation pouvant être provoquée par les chocs et de lui conférer par ailleurs une bonne aptitude au prélèvement.

En fait, l'obtention de ces propriétés implique par ailleurs que les autres composants de la composition cosmétique, et en particulier les charges qu'elle contient s'avèrent également appropriées pour une mise en forme de type poudre compacte.

Or, certaines charges sont précisément dénuées d'une bonne aptitude à être compactée. Elles sont encore qualifiées de charges « incompactables ». En conséquence leur présence dans une composition cosmétique destinée à être formulée sous forme de poudre compacte peut affecter significativement la cohésion de la poudre résultante, voire lorsqu'elles sont en quantité importante, rendre impossible l'obtention d'une poudre compacte correspondante au moyen d'une presse mécanique.

Les charges de faible densité peuvent également soulever ce type de problème pour la formulation de poudres compactes dotées de caractéristiques de cohésion et/ou de délitage satisfaisantes. Ainsi, lorsqu'une telle charge est présente en une teneur trop importante, généralement supérieure ou égale à 5 % en poids du poids total d'une composition destinée à être compactée, celle-ci ne peut être compactée de manière satisfaisante avec un liant classique tel que le stéarate d'isocétyle ou bien encore avec le liant siliconé décrit dans la demande WO 93/17660, utilisés à faible taux, notamment d'environ 3 à 5 % en poids. Or l'augmentation du taux de liant pour améliorer le compactage, par exemple à une échelle de 10 % en poids de liant dans la composition, s'avère généralement, pour sa part, préjudiciable à l'aspect de surface qui est alors trop gras. De plus, la forte cohésion ainsi obtenue rend difficile le délitage de la composition à l'aide d'un applicateur tel qu'une houppette ou un pinceau, ou bien au doigt.

Une autre alternative est illustrée par le document EP 0 717 979 de la société L'OREAL. Elle consiste à préparer des poudres qualifiées de compactes à partir d'environ 30 % en poids de charges dites incompactables environ 5 % en poids en liant liquide et plus de 50 % en eau par la technique de lyophilisation. Toutefois, ce procédé de lyophilisation est une technique lourde à mettre en oeuvre industriellement, notamment dans la mesure où il nécessite la congélation de la composition intermédiaire.

On connait par ailleurs les documents suivants.

Le document EP 1277 458, concerne l'utilisation d'agents gélifiants particuliers pour améliorer la douceur, la propriété émolliente, l'adhérence et la fraîcheur d'une composition.

Le document EP 0 132 631 vise à améliorer la cohésion de compositions comprenant de fortes concentrations en composés « perlescents ».

Le document FR 2 798 287 se rapporte à l'utilisation d'une charge particulière pour réduire la sensation huileuse à l'étalement des compositions comprenant des nacres.

Le document EP 1159 950 a pour objectif de limiter l'utilisation de cires dans les compositions cosmétiques.

Enfin, le document EP 0 657 486 concerne des compositions ayant une bonne tenue et une utilisation agréable grâce à une silicone spécifique.

Il existe donc un besoin pour une composition cosmétique de type poudre compacte qui bien que comprenant des quantités importantes en charges incompactables et/ou en charges de faible densité soit néanmoins pourvue de caractéristiques de cohésion et de délitage satisfaisantes, et ne soit pas par ailleurs contraignante en terme de préparation industrielle.

Les inventeurs ont constaté qu'il était possible de remédier aux inconvénients précités, sous réserve d'associer à ces charges incompactables et/ou à ces charges de faible densité une phase grasse de type liant de composition spécifique.

L'invention concerne une composition cosmétique sous forme de poudre compacte pour le maquillage et/ou le soin de la peau, notamment du visage, comprenant au moins :

- une phase pulvérulente,
- une phase grasse solide, et
- de 4 % à 15 % en poids par rapport au poids total de la compositions d'une phase grasse liquide,

ladite phase pulvérente comprenant plus de 5 % en poids par rapport au poids total de la composition d'au moins une charge incompactable et/ou de faible densité, à l'exclusion d'une composition comprenant 10 % en poids de cire de Carnauba, 6 % en poids de stéarate de magnésium et 5 % en poids de silicate de magnésium et d'aluminium amorphe par rapport au poids total de la composition.

Ladite composition cosmétique peut en outre être telle que,

- ladite phase grasse solide étant présente en une quantité telle que la poudre compacte possède une cohésion correspondant à une perte de masse évaluée au test de chute inférieure à 12 % en poids, et
- ladite phase pulvérulente étant telle que lorsqu'elle est mise en oeuvre à raison de 91,2 % en poids en mélange avec 8,8% en poids d'un liant liquide de référence
- tel que défini en exemple 1 et que l'ensemble est compacté à une pression de 2 x $10^7$ Pa (200 bars), il en résulte un compact dont la cohésion correspond à une perte de masse évaluée au test de chute supérieure à 12 % en poids et/ou un compact qui ne se délite pas de manière homogène.

Selon un autre de ses aspects, l'invention a pour objet l'utilisation soit d'une phase pulvérulente telle que définie ci-dessus, soit d'une charge ou d'un mélange de charges incompactables et/ou de faible densité en une teneur supérieure ou égale à 5 % en poids par rapport au poids total de la composition en association avec une phase grasse comprenant au moins une phase grasse solide, notamment telle que définie ci-dessus pour la préparation d'une composition cosmétique de type poudre compacte dotée de propriétés de cohésion et de délitage satisfaisantes.

Selon un autre de ses aspects, l'invention a pour objet un procédé de maquillage et/ou de soin de la peau comprenant au moins une étape d'application sur la peau d'au moins une composition selon l'invention.

Avantageusement, les compositions selon l'invention sont dotées de propriétés de cohésion et de délitage satisfaisantes. En d'autres termes, elles présentent une stabilité au stockage satisfaisante et en particulier ne s'altèrent pas (ou peu) lorsqu'elles subissent des chocs. Enfin, elles présentent également une bonne aptitude au prélèvement et à l'application.

<u>**Mesure de la cohésion et de l'aptitude au délitage**</u>

En ce qui concerne ces deux propriétés mécaniques, elles peuvent être mesurées, dans le cadre de la présente invention, selon les protocoles décrits ci-après.

Pour ce qui est de la phase pulvérulente, elle nécessite au préalable d'être formulée avec un liant dit liant liquide de référence.

La composition de ce liant, figurant également en exemple 1, est comme suit :

- 40,91 g de para-méthoxy-4 cinnamate d'éthyl-2 hexyle (Parsol MCX® de la société Roche Vitamins),
- 25 g de stéarate d'isocétyle,
- 25 g de polydiméthylsiloxane 10 cst (Fluid DC 200® 10 cst de Dow Corning), et
- 9,09 g du mélange* de conservateurs Phenonip® de la société Nipa (Clariant).

*Le mélange de conservateurs Phenonip® contient (% pondéral) :

- 70 % de phénoxy-2 éthanol,
- 17,4 % de p-hydroxybenzoate de méthyle,
- 4,2 % de p-hydroxybenzoate d'éthyle,
- 4,2 % de p-hydroxybenzoate de butyle,
- 2,1 % de p-hydroxybenzoate de propyle, et
- 2,1 % de p-hydroxybenzoate d'isobutyle.

Le mélange de liant liquide de référence (8,8 % en poids) et de la phase pulvérulente (91,2 % en poids) est placé dans une coupelle aluminium rectangulaire de dimensions 57/39/05 mm.

Ce mélange (10,5 g) est versé dans ladite coupelle puis une pression de 2 x $10^7$ Pa (200 bars) est appliquée.

Il est estimé que le produit ne se délite pas de manière homogène lorsque dès le frottement de la surface du produit, compacte dans la coupelle, avec les doigts ou avec une éponge sèche, par exemple une éponge rectangulaire telle que celle vendue sous la dénomination Yukilon Standard® par la société Yukigaya, celui-ci s'effrite, part en paquet et/ou se creuse. Le délitage du produit est alors considéré irrégulier et inhomogène sur toute la surface.

En ce qui concerne le test de chute, son protocole est comme suit.

Il consiste à faire subir au compact, placé à raison de 10,5 g dans une coupelle telle que définie ci-dessus, 10 chutes normalisées d'une hauteur de 20 cm sur une dalle en grès ayant une épaisseur d'au moins 30 mm ; le fond de

la coupelle est orienté vers la surface de réception de la dalle en grès. On mesure ensuite la perte en masse de produit puis on détermine le pourcentage de perte de masse de poudre.

Avantageusement, les compositions selon l'invention peuvent présenter une perte de masse au test de chute inférieure à 12 % en poids, notamment inférieure à 10 % en poids, voire inférieure à 8 % en poids, ceci traduisant de bonnes propriétés de cohésion.

Comme précisé précédemment, les compositions selon l'invention se présentent sous la forme d'une poudre compacte. Cet aspect compacté est obtenu en soumettant à une compression le mélange phase pulvérulente et liant gras associé.

Plus particulièrement, les compositions sous forme de poudre compacte conformes à l'invention peuvent être préparées en mélangeant l'ensemble des composants de la phase pulvérulente (charges et pigments) puis en ajoutant à ce mélange les composés constituant la phase grasse sous agitation. Le mélange est ensuite broyé, tamisé, puis versé dans une coupelle et compacté. Ce compactage est en général réalisé à l'aide d'une presse notamment en appliquant une pression de 50 à 250 bars pour obtenir la poudre compacte attendue.

Par conséquent, les compositions selon l'invention sont différentes des compositions pulvérulentes qualifiées de coulées, qui sont préparées par simple mélange d'une phase grasse à l'état fondu avec une phase pulvérulente.

Le fait que les compositions se présentent sous une forme compacte confère à celles-ci, comparativement à des compositions de type coulé, une meilleure aptitude à un délitage sous forme de particules solides « libres ».

Ainsi, on peut, à l'issu d'un décompactage d'une composition compacte selon l'invention, récupérer sa phase pulvérulente, ce qui n'est pas possible pour une composition coulée.

Ce décompactage peut notamment être réalisé selon le protocole suivant : on casse le produit compacté à l'aide d'une spatule au dessus d'un tamis (250μ) puis on tamise les paquets de poudre obtenus. On récupère ainsi la phase pulvérulente de la poudre compacte sous forme de particules solides « libres ».

**Phase pulvérulente**

La phase pulvérulente, au sens de l'invention, inclut tout composé solide et mélange de composés solides dont la température de fusion est bien supérieure à 250 °C.

Elle est généralement constituée d'au moins une charge et/ou d'au moins une matière colorante pulvérulente.

La charge peut être choisie parmi les charges incompactables, les charges de faible densité, les charges additionnelles, et leurs mélanges.

On entend par « charge additionnelle » toute charge distincte des charges incompactables et/ou des charges de faible densité décrites ci-après.

En ce qui concerne la matière colorante pulvérulente, elle peut notamment être choisie parmi les pigments et les nacres habituellement utilisés dans les compositions cosmétiques et/ou dermatologiques.

Selon une variante, la charge est une poudre traitée ou enrobée hydrophobe.

Par « poudre traitée ou enrobée hydrophobe », on entend une poudre traitée en surface avec un agent hydrophobe, notamment tel que décrit ci-après.

A titre illustratif, cet agent hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbone, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyle mentionné dans les composés cités précédemment désigne notamment un groupe alkyle ayant de 1 à 30 atomes de carbone, de préférence ayant de 5 à 16 atomes de carbone.

Des pigments ou des charges traités hydrophobes sont notamment décrits dans la demande EP-A-1086683.

La phase pulvérulente de la composition selon l'invention peut comprendre une poudre ou un mélange de poudres traitées hydrophobe en une teneur supérieure ou égale à 50 % en poids, notamment supérieure ou égale à 60 % en poids, voire supérieure ou égale à 70 % en poids par rapport au poids total de la phase pulvérulente.

Comme précédemment décrit, la composition selon l'invention peut comprendre au moins une charge incompactable et/ou au moins une charge de faible densité.

Charges incompactables

Selon une variante de l'invention, la composition comprend une charge incompactable ou un mélange de charges incompactables en une teneur supérieure ou égale à 5 % en poids par rapport au poids total de la composition.
Par « charge incompactable », on entend au sens de la présente invention une poudres qui, à partir d'une certaine quantité qui dépendra de la matière en question, notamment à partir d'une teneur supérieure ou égale à 5 % en poids par rapport au poids total de la composition n'a pas une bonne aptitude à être compactée au moyen d'une presse manuelle, notamment sous une pression de 2 x $10^7$ Pa (200 bars) et/ou nuit à l'obtention de produits ayant une bonne résistance aux chocs, notamment ayant une perte de masse supérieure à 12 % au test de chute, et/ou ne permet pas d'obtenir de produit compact apte à se déliter de manière homogène.
La charge incompactable peut être de type organique ou minéral, de forme sphérique ou lamellaire.
Parmi les charges incompactables de forme sphérique on peut notamment citer :

- les microsphères de silice, notamment à porosité ouverte, et en particulier les microsphères de silice creuses, telles que les 'SILICA BEADS SB 700/HA®' ou 'SILICA BEADS SB 700®' de la société MAPRECOS, les 'SUNSPHERES H-33®' et les 'SUNSPHERES H-51®' de la société ASAHI GLASS, ces microsphères peuvent, le cas échéant, être imprégnées d'un actif cosmétique,
- les microsphères microporeuses de polymères, qui ont une structure analogue à celle d'une éponge ; elles ont, en général, une surface spécifique d'au moins 0,5 $m^2$/g et en particulier d'au moins 1 $m^2$/g, ladite surface spécifique n'ayant pas de limite supérieure autre que celle résultant de la possibilité pratique de réaliser des microsphères de porosité très élevée : la surface spécifique peut, par exemple, atteindre 1 000 $m^2$/g ou même davantage. A titre illustratif de ces microsphères, on peut citer les microsphères de polymères acryliques, telles que celles en copolymère d'acrylate réticulé 'POLYTRAP 6603 ADSORBER®' de la société RP SCHERRER, et celles de polyméthacrylate de méthyle 'MICROPEARL M 100®' de la société SEPPIC,
- la poudre de polyuréthane telle que la poudre de copolymère de diisocyanate d'hexaméthylène et de triméthylol hexyl lactone vendue sous la dénomination « PLASTIC POWDER D-400® » par la société Toshiki,
- les microcapsules de polymères qui comportent une seule cavité fermée et forment un réservoir, qui peut contenir un liquide, notamment un actif cosmétique ; elles sont préparées par des procédés connus tels que ceux décrits dans le brevet US-A 3 615 972 et EP-A 0 56219. Elles peuvent être réalisées, par exemple, en polymères ou copolymères d'acides, d'amines ou d'esters monomères à insaturation éthylénique, en polymères urée-formaldéhyde, en polymères ou copolymères de chlorure de vinylidène; à titre d'exemple, on peut citer les microcapsules faites de polymères ou copolymères d'acrylate ou de méthacrylate de méthyle, ou encore de copolymères de chlorure de vinylidène et d'acrylonitrile, comme l' « EXPANCEL® » de la société EXPANCEL ; parmi ces derniers, on indiquera, notamment, ceux qui contiennent, en poids 20-60% de motifs dérivés de chlorure de vinylidène, 20-60% en poids de motifs dérivés d'acrylonitrile et 0-40% en poids d'autres motifs tels que des motifs dérivés d'un monomère acrylique et/ou styrénique; on peut également utiliser des polymères ou copolymères acryliques réticulés,
- les poudres d'organopolysiloxane réticulés élastomères, notamment décrites dans le document JP-A-02-243612, telles que celles vendues sous la dénomination «TREFIL POWDER E-506C» par la société DOW CORNING, les poudres d'organopolysiloxane réticulé élastomère enrobée de résine de silicone, notamment de résine silsesquioxane, comme décrit par exemple dans le brevet US5538793. De tels élastomère sont vendus sous les dénomination "KSP-100", "KSP-101", "KSP-102", "KSP-103", KSP-104", "KSP-105" par la société Shin Etsu, et
- leurs mélanges.

Charges de faible densité

Selon une autre variante de l'invention, la composition comprend une charge de faible densité ou un mélange de charges de faible densité en une teneur d'au moins 5 % en poids par rapport au poids total de la composition.
Par « charge de faible densité » on entend au sens de la présente invention des charges dont la densité non-tassée, mesurée par le protocole donné ci-après, varie de 0,01 à 0,50 g/$cm^3$, notamment allant de 0,05 à 0,50 g/$cm^3$, et en particulier allant de 0,10 à 0,50 g/$cm^3$.

Protocole de mesure de la densité non-tassée ($D_{NT}$) :

Dans une éprouvette graduée de 250 ml ayant une masse $M_0$ (en g) on verse, à l'aide d'un entonnoir, un volume de poudre compris entre 240 et 250 ml. On lit ensuite sur l'éprouvette le volume $V_0$ (en $cm^3$) de poudre versée et on pèse l'éprouvette remplie de poudre pour mesurer la masse $M_1$ (en g).
La densité non-tassée est déterminée selon la relation suivante :

$$D_{NT} = (M_1 - M_0) / V_0$$

Lesdites charge de faible densité peuvent également être caractérisées par une densité tassée, mesurée par le protocole donné ci-après, allant de 0,01 à 0,80 g/cm$^3$, notamment allant de 0,05 à 0,80 g/cm$^3$, et en particulier allant de 0,10 à 0,80 g/cm$^3$.

Protocole de mesure de la densité tassée ($D_T$) :

Dans une éprouvette graduée de 250 ml ayant une masse $M_0$ (en g), on verse, à l'aide d'un entonnoir, un volume de poudre compris entre 240 et 250 ml. On lit ensuite sur l'éprouvette le volume $V_0$ (en cm$^3$) de poudre versée et on pèse l'éprouvette remplie de poudre pour mesurer la masse $M_1$ (en g).

Puis on place l'éprouvette sur l'appareil STAV 2003® de chez Stampf Volumeter. L'éprouvette est ensuite soumise à une série de 2 500 tassements, puis on mesure à la fin de chaque série le volume $V_n$ (volume après la n$^{ième}$ série de 2 500 tassements) de la poudre dans l'éprouvette.

Dès que $V_n - V_{n+1} =< (2 \times V_n) / 100$, on arrête le tassement de l'éprouvette et on note le volume $V_n$ (en cm$^3$).

La densité tassée est déterminée selon la relation suivante :

$$D_T = (M_1 - M_0) / V_n$$

La charge de faible densité peut être choisie dans la liste des charges incompactables données ci-dessus. Plus particulièrement, elle peut être choisie parmi les charges décrites dans le tableau suivant :

| Charges de faible densité | Densité non-tassée (g/cm$^3$) | Densité tassée (g/cm$^3$) |
|---|---|---|
| Sunsphère H-33® de la société Asahi Glass | 0,10 | 0,14 |
| Trefil Powder E-506C® de la société Dow Corning | 0,19 | 0,27 |
| KSP-100® de la société Shin Etsu | 0,27 | 0,39 |
| Silica Beads SB 700® de la société Maprecos | 0,28 | 0,48 |
| Sunsphère H-51® de la société Asahi Glass | 0,28 | 0,49 |
| Micropearl M-100® de la société SEPPIC | 0,39 | 0,64 |
| Plastic Powder D-400® de la société Toshild | 0,46 | 0,76 |
| Polytrap 6603 Adsorber® de la société RP Scherrer | 0.05 à 0,12 | 0,07 à 0,10 |

La composition selon l'invention peut comprendre une charge ou un mélange de charges incompactables et/ou de charges de faible densité en une teneur variant de 5 à 15 % en poids, notamment de 5 à 13 % en poids, et en particulier de 5 à 10 % en poids par rapport au poids total de la composition.

La phase pulvérulente peut également comprendre au moins une charge additionnelle. Celles-ci peuvent être minérales ou organiques. Ces charges peuvent être des particules de toute forme, notamment plaquettaires, sphériques ou oblongues, quelle que soit leur forme cristallographique (par exemple feuillet, cubique, hexagonale, orthorhombique, etc...). Parmi les charges additionnelles utilisables dans les compositions selon l'invention, on peut notamment citer le talc, le mica, la silice, le kaolin, les poudres de polyamide (Nylon®), de poly-β-alanine et de polyéthylène, les poudres de polymères de tétrafluoroéthylène (Téflon®), la lauroyl-lysine, l'amidon, le nitrure de bore, les poudres de polymères d'acide acrylique, les microbilles de résine de silicone (Tospearls® de Toshiba, par exemple), le carbonate de calcium précipité, le carbonate et l'hydro-carbonate de magnésium, l'hydroxyapatite, les microsphères de silice creuses, les microcapsules de verre ou de céramique.

Cette charge additionnelle ou mélange de charges additionnelles peut être présent en une teneur variant de 40 à 95 % en poids, notamment de 45 à 85 % en poids, et en particulier de 45 à 70 % en poids par rapport au poids total de la composition.

Comme précisé précédemment, les compositions selon l'invention peuvent également comprendre au moins une matière colorante pulvérulente choisie parmi les pigments et les nacres.

Les pigments peuvent être blancs ou colorés, minéraux et/ou organiques, enrobés ou non. On peut notamment

citer parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut notamment citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Cette matière colorante pulvérulente peut généralement être présente dans la composition en une teneur variant de 0,5 à 30 % en poids, notamment de 1 à 22 % en poids, et en particulier de 3 à 18 % en poids par rapport au poids total de la composition.

**Phase grasse :**

Comme précisé précédemment la phase grasse est encore communément appelée « liant » et sert notamment de milieu dispersant pour la phase particulaire. Cette phase grasse peut être présente dans la composition selon l'invention en une teneur allant de 12 à 35 % en poids, et notamment de 15 à 30 % en poids par rapport au poids total de la composition.

Phase grasse solide :

La composition selon l'invention comporte au moins une phase grasse comprenant au moins une phase grasse solide, dite encore « liant solide » dans un rapport pondéral phase grasse solide / charge(s) incompactable(s) et/ou charge(s) de faible densité supérieur ou égal à 1, et notamment supérieur ou égal à 1,5.

Par « liant solide », on entend au sens de la présente invention une phase grasse dont le point de fusion peut être supérieur ou égal à 30 °C, notamment peut aller de 30 à 250 °C et en particulier de 30 à 230 °C.

Cette phase grasse solide peut comprendre au moins un composé choisi parmi les cires, les savons métalliques et leurs mélanges.

Par "cire", au sens de la présente invention, on entend un composé gras lipophile, solide à température ambiante (25°C) et pression atmosphérique (760 mm Hg, soit $10^5$ Pa), à changement d'état solide/liquide réversible, ayant en particulier une température de fusion supérieure ou égale à 30°C, notamment supérieure ou égale à 55 °C, et pouvant aller jusqu'à 250 °C, notamment jusqu'à 230 °C, et en particulier jusqu'à 120 °C.

En portant la cire à sa température de fusion, il est possible de la rendre miscible aux huiles et de former un mélange homogène microscopiquement, mais en rétablissant la température du mélange à la température ambiante, on obtient une recristallisation de la cire dans les huiles du mélange.

Les valeurs de point de fusion correspondent, selon l'invention, au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.

Les cires, au sens de l'invention, peuvent être celles utilisées généralement dans les domaines cosmétiques ou dermatologiques. Elles peuvent notamment être hydrocarbonées, siliconées et/ou fluorées, comportant éventuellement des fonctions ester ou hydroxyle. Elles peuvent être également d'origine naturelle ou synthétique.

A titre illustratif et non limitatif de ces cires, on peut notamment citer :

- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55 °C,
- les cires obtenues par hydrogénation catalytique d'huiles animales ou végétales ayant des chaînes grasses, linéaires ou ramifiées, en $C_8$-$C_{32}$, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées, et
- leurs mélanges

Selon une variante particulière de l'invention, la phase grasse solide peut comprendre au moins une cire choisie parmi les cires de carnauba, les cires de paraffine et leurs mélanges.

Selon une autre variante particulière, la phase grasse solide peut comprendre au moins une cire présente totalement ou partiellement sous forme de poudre, notamment micronisée, pour faciliter sa mise en oeuvre dans la

préparation de la composition cosmétique.

Parmi les cires utilisables sous forme de poudre, on peut notamment citer les microbilles de cire de Carnauba vendues sous la dénomination Microcare 350® par la société Micro Powders et les microbilles de cire de paraffine vendues sous la dénomination Microease 114S® par la société Micro Powders.

Le liant solide peut également être choisi parmi les savons métalliques.

Parmi ceux ci, on peut notamment citer les savons métalliques d'acides gras ayant de 12 à 22 atomes de carbone, et en particulier ceux ayant de 12 à 18 atomes de carbone.

Le métal du savon métallique peut notamment être du zinc ou du magnésium.

L'acide gras peut notamment être choisi parmi l'acide laurique, l'acide myristique, l'acide stéarique, l'acide palmitique.

Comme savon métallique, on peut utiliser le laurate de zinc, le stéarate de magnésium, le myristate de magnésium, le stéarate de zinc, et leurs mélanges.

Selon une variante particulière de l'invention, la phase grasse solide peut comprendre au moins un savon métallique présent totalement ou partiellement sous forme de poudre.

La phase grasse solide peut être présente en une teneur supérieure ou égale à 35 % en poids, notamment supérieure ou égale à 40 % en poids, et en particulier supérieure ou égale à 50 % en poids par rapport au poids total de la phase grasse.

Le rapport pondéral phase grasse solide / charge(s) incompactable(s) ou charge(s) de faible(s) densité(s) de la composition selon l'invention peut aller de 1 à 4, notamment de 1 à 3, et en particulier de 1 à 2,5.

La composition selon l'invention peut comprendre au moins une phase grasse solide en une teneur variant de 8 à 25 % en poids, notamment de 10 à 22 % en poids, et en particulier de 10 à 20 % en poids par rapport au poids total de la composition.

<u>Phase grasse liquide :</u>

La phase grasse de la composition selon l'invention peut en outre comprendre une phase grasse liquide comportant au moins une huile. Cette huile peut être choisie parmi les huiles utilisées classiquement comme liant dans les poudres compactes.

Ces huiles peuvent être notamment choisies parmi :

- l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures, telles que les huiles de paraffine, le squalane, la vaseline, le polydécène, notamment hydrogéné, comme le « CERAFLOW E® » commercialisé par la société SHAMROCK ;
- les esters gras, tels que le myristate d'isopropyle, le palmitate d'isopropyle, le stéarate de butyle, le stéarate d'isodécyle, le laurate d'hexyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de 2-diéthylhexyle, le malate de diisostéaryle, le triisostéarate de glycérine ou de diglycérine ;
- les huiles de silicone telles que les polyméthylsiloxanes, les polyméthylphénylsiloxanes, les polysiloxanes modifiés par des acides gras, des alcools gras ou des polyoxyalkylènes, les silicones fluorées ou les huiles perfluorées ;
- les acides gras supérieurs tels que l'acide myristique, l'acide palmitique, l'acide stéarique, l'acide béhénique, l'acide oléique, l'acide linoléique, l'acide linolénique ou l'acide isostéarique;
- les alcools gras supérieurs tels que le cétanol, l'alcool stéarylique ou l'alcool oléique ;
- les poly méthylfluoroalkyl diméthylsiloxanes de formule (I) :

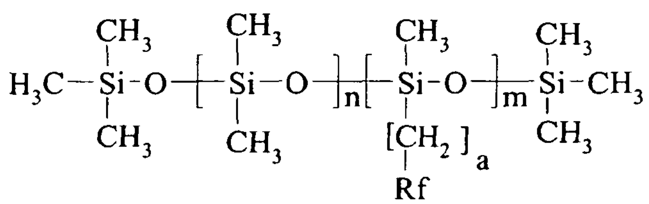

(I)

dans laquelle :

- n représente un entier variant de 5 à 90, notamment de 30 à 80 et en particulier de 50 à 80,
- m représente un entier variant de 1 à 150, notamment de 1 à 80, et en particulier de 1 à 40,
- a représente un entier variant de 0 à 5, et
- Rf désigne un radical perfluoroalkyle comprenant de 1 à 8 atomes de carbone ; et
- leurs mélanges.

Comme composés de formule (I) convenant tout particulièrement à l'invention, on peut notamment citer ceux vendus sous la dénomination X22-819®, X22-820®, X22-821®, X22-822® par la société SHIN-ETSU.
La composition selon l'invention peut comprendre en outre une phase grasse liquide en une teneur variant de 4 à 15 % en poids, notamment de 6 à 13 % en poids, et en particulier de 7 à 13 % en poids par rapport au poids total de la composition.
Selon une variante particulière, la phase grasse liquide et la ou les charge(s) incompactable(s) ou charge(s) de faible densité sont présentes dans les compositions selon l'invention en un rapport pondéral inférieur ou égal à 3, notamment inférieur ou égal à 2,5, en particulier inférieur ou égal à 2, voire inférieur ou égal à 1,5.
Selon une variante particulière la composition selon l'invention peut être exempte de phase grasse liquide.
Bien entendu, l'homme de l'art veillera à ajuster les quantités de phase grasse solide et éventuellement liquide de la composition selon l'invention de manière telle que les propriétés attendus en termes de cohésion et d'effet matifiant dans le temps soient satisfaisantes.

**Additifs :**

La composition peut également comprendre d'autres ingrédients (adjuvants) usuellement utilisés en cosmétique tels que les agents colorants hydrosolubles ou liposolubles, les conservateurs, les actifs cosmétiques, les agents hydratants, les filtres UV, les épaississants, l'eau, les tensioactifs et/ou les parfums.
Bien entendu, l'homme de l'art veillera à choisir le ou les éventuels adjuvants ajoutés à la composition selon l'invention de manière telle que les propriétés avantageuses attachées intrinsèquement à la composition conforme à l'invention ne soient pas, ou substantiellement pas, altérées par l'addition envisagée.
La composition selon l'invention peut notamment se présenter sous la forme d'un produit de maquillage de type poudre compact, en particulier un fard à joues, un fard à paupières, une poudre pour le visage, un fond de teint, un produit anti-cernes, un produit de maquillage du corps, ou bien encore sous la forme d'un produit de soin pour le visage, d'un produit de soin du corps.
Les exemples de compositions ci-après sont donnés à titre illustratif et sans caractère limitatif de l'invention.

Exemple 1 : liant liquide de référence

- para-méthoxy-4 cinnamate d'éthyl-2 hexyle
  (Parsol® MCX de la société Roche Vitamins), 40,91 g
- stéarate d'isocétyle, 25 g
- polydiméthylsiloxane 10 cst (Fluid DC 200® 10 cst de Dow Corning), et 25 g
- mélange* de conservateurs Phenonip® de la société Nipa (Clariant) 9,09 g

*Le mélange de conservateurs Phenonip® contient :

- phénoxy-2 éthanol, 70 g
  p-hydroxybenzoate de méthyle, 17,40 g
- p-hydroxybenzoate d'éthyle, 4,20 g
- p-hydroxybenzoate de butyle, 4,20 g
- p-hydroxybenzoate de propyle, et 2,10 g
- p-hydroxybenzoate d'isobutyle 2,10 g

Exemple 2 : Fond de teint

a) Composition selon l'invention

- Oxydes de fer jaune, brun, noir 3 ,23 g
- Microsphères de silice amorphe : Sunsphère® H-33 de Asahi — Glass 1,00 g
- Conservateurs qs
- Dioxyde de titane 10,55 g
- Sericite 15,00 g
- Nitrure de bore 6,00 g
- Talc 28,30 g
- Poudre de polyuréthane et de silice (98/2) : Plastic Powder® D-400 de Toshiki 3,00 g
- Dioxyde de titane — oxyde de zinc — talc enrobé méthylhydrogéno polysiloxane : TZ Powder® type 2 de Myoshi 4,00 g
- Poudre de polydimethylsiloxane réticulé : Trefil Powder® E-506 C de Dow Corning 2,00 g
- Poudre de copolymère éthylène/acide acrylique : Flobeads® EA-209 de Sumitomo 5,00 g
- Microbilles de cire de paraffine : Microease® 114S de Micro Powders 10,00 g
- Stéarate de magnésium 3,00 g
- Polyméthyl-trifluoro-propyl diméthylsiloxane : X22-819® de Shin Etsu 2,92 g
- P-méthoxy-4-cinnamate d'éthyl-2-hexyle 4,00 g
- Isononanoate d'isononyle 1,20 g

La composition est préparée en mélangeant l'ensemble des poudres puis en y ajoutant la phase grasse, ce mélange étant ensuite broyé et tamisé jusqu'à l'obtention d'un mélange homogène. Une partie de la composition (10,5 g) est placée dans une coupelle puis compactée à une pression de 2 x $10^7$ Pa (200 bars).

La composition a une cohésion telle que la perte de produit lors du test de chute est de 2 % en poids.

L'application sur le visage de la poudre compacte procure un maquillage présentant une bonne homogénéité de la couleur et de la matité au cours de la journée.

b) 91,2 % en poids de la même phase pulvérulente est formulée en présence de 8,8 % en poids d'un liant liquide, à savoir 3,60 g de P-méthoxy-4-cinnamate d'éthyl-2-hexyle, 2,20 g de polydiméthyl siloxane, et 2,20 g de stéarate d'isocétyle et 0,80 g d'un mélange de conservateurs :

- Oxydes de fer jaune, brun, noir 3,77 g
- Microsphères de silice amorphe : Sunsphère® H-33 de Asahi — Glass 1,17 g
- Conservateurs qs
- Dioxyde de titane 12,32 g
- Sericite 17,52 g
- Nitrure de bore 7,01 g
- Talc 33,06 g
- Poudre de polyuréthane et de silice (98/2) : Plastic Powder® D-400 de Toshiki 3,50 g
- Dioxyde de titane — oxyde de zinc — talc enrobé méthylhydrogéno polysiloxane : TZ Powder® type 2 de Miyoshi 4,67 g
- Poudre de polydimethylsiloxane éticulé : Trefil Powder® E-506 C de Dow Corning 2,34 g
- Poudre de copolymère éthylène/acide acrylique : Flobeads® EA-209 de Sumitomo 5,84 g
- P-méthoxy-4-cinnamate d'éthyl-2-hexyle 3,60 g
- Stéarate d'isocétyle 2,20 g
- polydiméthylsiloxane 10 cst (Fluid DC® 200 10 est de Dow Corning) 2,20 g
- Mélange de conservateurs Phenonip® de la société Nipa (Clariant) 0,80 g

La composition compactée a été testée pour évaluer ses propriétés mécaniques. La perte de produit lors du

test de chute est de 17,6 % en poids ce qui reflète un défaut de cohésion. En outre, le délitage du produit compacté avec une éponge sèche s'avère, pour sa part, trop important dans la mesure où le produit s'effrite dès le contact avec l'éponge et le compact se creuse. Le liant liquide ne permet donc pas d'obtenir une poudre compacte satisfaisante.

Exemple 3 : Fond de teint compact

a) Composition selon l'invention

- Oxydes de fer jaune, brun, noir 5,75 g
- Talc 56,25 g
- Microsphères de silice amorphe : Sunsphère® H-33 de Asahi Glass 10,00 g
- Microbilles de cire de carnauba : Microcare® 350 de Micro Powders 20,00 g
- Polydécène hydrogéné : Ceraflow E® de Shamrock 3,00 g
- Polyméthyl-trifluoro-propyl diméthylsiloxane : X22-819® de Shinetsu 5,00 g

La composition est préparée en mélangeant l'ensemble des poudres (oxydes de fer, talc, silice, cire de carnauba) puis en y ajoutant les huiles (polydécène hydrogéné, silicone fluorée), ce mélange est ensuite broyé et tamisé jusqu'à l'obtention d'un mélange homogène. Une partie de la composition (10,5 g) est placée dans une coupelle puis compactée à une pression de 2 x $10^7$ Pa (200 bars).

La composition compactée a une cohésion telle que la perte de produit lors du test de chute est inférieure à 5,72 % en poids.

On obtient une poudre compacte dont l'application sur le visage procure un maquillage présentant une bonne matité au cours de la journée.

b) 91,2 % en poids de la même phase pulvérulente est formulée en présence de 8,8 % en poids d'un liant liquide, à savoir 3,60 g de P-méthoxy-4-cinnamate d'éthyl-2-hexyle, 2,20 g de polydiméthyl siloxane, et 2,20 g de stéarate d'isocétyle et 0,80 g d'un mélange de conservateurs :

- Oxydes de fer jaune, brun, noir 7,27 g
- Talc 71,26 g
- Microsphères de silice amorphe : Sunsphère® H-33 de Asahi Glass 12,67 g
- P-méthoxy-4-cinnamate d'éthyl-2-hexyle 3,60 g
- Stéarate d'isocétyle 2,20 g
- polydiméthylsiloxane 10 cst (Fluid DC® 200 10 cst de Dow Corning) 2,20 g
- Mélange de conservateurs Phenonip® de la société Nipa (Clariant) 0,80 g

La composition est préparée de la même manière que décrite en a).

La composition compactée a une cohésion telle que la perte de produit lors du test de chute est supérieure à 12 % en poids. Le délitage du produit compacté est trop important. Le produit s'effrite dès le contact avec une éponge sèche et le compact se creuse. Le liant liquide ne permet donc pas d'obtenir une poudre compacte donnant satisfaction en terme de cohésion et de délitage.

Exemple 4 : Fond de teint

**a) Composition selon l'invention**

- Oxydes de fer jaune, brun, noir 3,23 g

- Dioxyde de titane enrobé de lauroyl lysine (95/5) 9,00 g

- Dioxyde de titane — oxyde de zinc — talc enrobé méthylhydrogénopolysiloxane : TZ Powder® type 2 de Miyoshi 4,00 g
- Conservateurs qs
- Talc enrobé (SNI TA 68R® de Miyoshi) 30,80 g
- Microsphères de silice amorphe :
  Sunsphère® H-33 de Asahi — Glass 1,00 g
- Microbilles de silice :
  SILICA BEADS® SB150 de Myoshi 1,00 g
- Poudre de polyuréthane et de silice (98/2) :
  Plastic Powder® D-400 de Toshiki 5,00 g
- Poudre de copolymère éthylène/acide acrylique :
  Flobeads® EA-209 de Sumitomo 5,00 g
- Poudre de nylon :
  ORGASOL® 2002 EXTRA D NAT COS 5,00 g
- Poudre de polydimethylsiloane réticulé :
  Trefil Powder® E-506 C de Dow Corning 2,00 g
- Microbilles de cire de paraffine :
  Microease® 114S de Micro Powders 8,00 g
- Séricite enrobé de cire de Carnauba (94,5/5,5) :
  Séricite UW-A5® de Toshiki 15,00 g
- Stéarate de magnésium 3,00 g
- Isononanoate d'Isononyle 1,20 g
- Polyméthyl-trifluoro-propyl diméthylsiloxane :
  X22-819® de Shin Etsu 2,00 g
- P-méthoxy-4-cinnamate d'éthyl-2-hexyle 4,00 g

La composition est préparée en mélangeant l'ensemble des poudres (oxydes de fer, silice, polyuréthane...) puis en y ajoutant les huiles (isononanoate d'isononyle, silicone fluorée, P-méthoxy-4-cinnamate d'éthyl-2-hexyle), ce mélange est ensuite broyé et tamisé jusqu'à l'obtention d'un mélange homogène. Une partie de cette composition (10,5 g) est placée dans une coupelle puis compactée à une pression de 2 x $10^7$ Pa (200 bars).

La composition compactée a une cohésion telle que la perte de produit lors du test de chute est de 2,2 % en poids.

On obtient une poudre compacte dont l'application sur le visage procure un maquillage présentant une bonne tenue en matité au cours de la journée.

b) 91,2 % en poids de la même phase pulvérulente est formulée en présence de 8,8 % en poids d'un liant liquide, à savoir 3,60 g de P-méthoxy-4-cinnamate d'éthyl-2-hexyle, 2,20 g de polydiméthyl siloxane, et 2,20 g de stéarate d'isocétyle et 0,80 g d'un mélange de conservateurs :

- Oxydes de fer jaune, brun, noir 3,64 g
- Dioxyde de titane enrobé de lauroyl lysine (95/5) 10,13 g
- Dioxyde de titane — oxyde de zinc — talc enrobé
  méthylhydrogéno polysiloxane : TZ Powder® type 2 de Miyoshi 4,50 g
- Conservateurs qs
- Talc enrobé (SNI TA 68R® de Miyoshi) 34,65 g
- Microsphères de silice amorphe :

Sunsphère® H-33 de Asahi — Glass 1,13 g

- Microbilles de silice :
  SILICA BEADS® SB150 de Myoshi 1,12 g
- Poudre de polyuréthane et de silice (98/2) :
  Plastic Powder® D-400 de Toshiki 5,63 g
- Poudre de copolymère éthylène/acide acrylique :
  Flobeads® EA-209 de Sumitomo 5,63 g
- Poudre de nylon :
  ORGASOL® 2002 EXTRA D NAT COS 5,63 g
- Poudre de polydimethylsiloane réticulé :
  Trefil Powder E-506 C de Dow Corning 2,25 g
- Séricite enrobé de cire de Carnauba (94,5/5,5) :
  Séricite UW-A5® de Toshiki 16,89 g
- P-méthoxy-4-cinnamate d'éthyl-2-hexyle 3,60 g
- Stéarate d'isocétyle 2,20 g
- polydiméthylsiloxane 10 cst (Fluid DC® 200 10 cst de Dow Corning) 2,20 g
- Mélange de conservateurs Phenonip® de la société Nipa (Clariant) 0,80 g

La composition compactée a une cohésion telle que la perte de produit lors du test de chute est de 2,38 % en poids.

Cependant, le délitage du produit compacté est trop important, le produit s'effrite dès le contact avec l'éponge et le compact se creuse. Le liant liquide ne permet donc pas d'obtenir une poudre compacte présentant un délitage satisfaisant.

## Revendications

**1.** Composition cosmétique sous forme de poudre compacte pour le maquillage et/ou le soin de la peau, notamment du visage, comprenant au moins :

- une phase pulvérulente,
- une phase grasse solide, et
- de 4 % à 15 % en poids par rapport au poids total de la composition d'une phase grasse liquide,

ladite phase pulvérulente comprenant plus de 5 % en poids par rapport au poids total de la composition d'au moins une charge incompactable et/ou de faible densité,
à l'exclusion d'une composition comprenant 10 % en poids de cire de Carnauba, 6 % en poids de stéarate de magnésium et 5 % en poids de silicate de magnésium et d'aluminium amorphe par rapport au poids total de la composition.

**2.** Composition selon la revendication 1 dans laquelle :

- ladite phase grasse solide est présente en une quantité telle que la poudre compacte possède une cohésion correspondant à une perte de masse, évaluée au test de chute, inférieure à 12 % en poids, et
- ladite phase pulvérulente est telle que lorsqu'elle est mise en oeuvre à raison de 91,2 % en poids en mélange avec 8,8 % d'un liant liquide de référence, à savoir une composition contenant 40,91 g de para-méthoxy-4-cinnamate d'éthyl-2-hexyle, 25 g de stéarate d'isocétyle, 25 g de polydiméthylsiloxane 10 cst, et 9,09 g d'un mélange de conservateur contenant 70 % de phenoxy-2-éthanol, 17,4 % de p-hydroxybenzoate de méthyle, 4,2 % de p-hydroxybenzoate d'éthyle, 4,2 % de p-hydroxybenzoate de butyle, 2,1 % de p-hydroxybenzoate de propyle et 2,1 % de p-hydroxybenzoate d'isobutyle, et que l'ensemble est compacté à une pression de $2 \times 10^7$ Pa, il en résulte un compact dont la cohésion correspond à une perte de masse au test de chute supérieure à 12 % en poids et/ou un compact qui ne se délite pas de manière homogène.

**3.** Composition selon l'une quelconque des revendications précédentes, **caractérisée en ce que** ladite phase pulvérulente comprend en outre au moins une matière colorante pulvérulente.

4. Composition selon l'une quelconque des revendications précédentes, **caractérisée en ce que** la charge incompactable est choisie parmi les charges incompactables de type minéral, de type organique et leurs mélanges.

5. Composition selon l'une quelconque des revendications précédentes, **caractérisée en ce que** la charge incompactable est choisie parmi les microsphères de silice, les microsphères microporeuses de polymères, la poudre de polyuréthane, les microcapsules de polymères comportant une seule cavité fermée, les poudres d'organopolysiloxanes réticulés élastomères et leurs mélanges.

6. Composition selon l'une quelconque des revendications précédentes, **caractérisée en ce que** la charge incompactable est choisie parmi les microsphères de silice à porosité ouverte, les microsphères de silice creuses, les microsphères microporeuses de polymères acryliques, la poudre de copolymère de diisocyanate d'hexaméthylène et de triméthylol hexyl lactone, les microcapsules de polymères ou copolymères d'acrylate, de méthacrylate de méthyle, ou de copolymères de chlorure de vinylidène et d'acrylonitrile, les poudres d'organopolysiloxane réticulé élastomère enrobé de résine silsesquioxane.

7. Composition selon l'une quelconque des revendications précédentes, **caractérisée en ce que** la charge de faible densité présente une densité non-tassée allant de 0,05 à 0,50 g/$cm^3$, et en particulier allant de 0,10 à 0,50 g/$cm^3$.

8. Composition selon la revendication précédentes, **caractérisée en ce que** la charge de faible densité présente une densité tassée allant de 0,01 à 0,80 g/$cm^3$, notamment allant de 0,05 à 0,80 g/$cm^3$, et en particulier allant de 0,10 à 0,80 g/$cm^3$.

9. Composition selon l'une quelconque des revendications précédentes, **caractérisée en ce que** ladite charge de faible densité est choisie parmi les charges citées en revendication 6 à 8.

10. Composition selon l'une quelconque des revendications précédentes, **caractérisée en ce que** ladite charge de faible densité ou incompactable, ou un mélange desdites charges est présent(e) en une teneur allant de 5 à 15 % en poids, notamment de 5 à 13 % en poids, et en particulier de 5 à 10 % en poids par rapport au poids total de la composition.

11. Composition selon l'une quelconque des revendications précédentes, **caractérisée en ce que** la phase grasse solide comprend au moins un composé choisi parmi les cires, les savons métalliques et leurs mélanges.

12. Composition selon l'une quelconque des revendications précédentes, **caractérisée en ce que** la phase grasse solide .comprend au moins un composé choisi parmi :

- la cire d'abeilles, la cire de lanoline, et les cires d'insectes de Chine ; la cire de riz, la cire de Carnauba, la cire de Candellila, la cire d'Ouricury, la cire de fibres de liège, la cire de canne à sucre, la cire du Japon et la cire de sumac; la cire de montan, les cires microcristallines, les cires de paraffine, les ozokérites, la cire de cérésine, la cire de lignite, les cires de polyéthylène, les cires obtenues par la synthèse de Fisher-Tropsch, les esters d'acides gras et les glycérides concrets à 40 °C et notamment à plus de 55°C,
- les cires obtenues par hydrogénation catalytique d'huiles animales ou végétales ayant des chaînes grasses, linéaires ou ramifiées, en $C_8$-$C_{32}$, notamment l'huile de jojoba hydrogénée, l'huile de tournesol hydrogénée, l'huile de ricin hydrogénée, l'huile de coprah hydrogénée et l'huile de lanoline hydrogénée,
- les cires de silicones ou les cires fluorées,
- le laurate de zinc, le stéarate de magnésium, le myristate de magnésium, le stéarate de zinc, et
- leurs mélanges.

13. Composition selon l'une quelconque des revendications précédentes, **caractérisée en ce que** la phase grasse solide comprend au moins un composé choisi parmi les cires de paraffine, les cires de carnauba et leurs mélanges.

14. Composition selon l'une quelconque des revendications précédentes, **caractérisée en ce que** la phase grasse solide comprend au moins une cire au moins partiellement sous forme de poudre, telle que les microbilles de cire de paraffine et les microbilles de cire de carnauba.

15. Composition selon l'une quelconque des revendications précédentes, **caractérisée en ce que** la phase grasse solide comprend au moins un savon métallique choisi parmi le laurate de zinc, le stéarate de magnésium, le myristate de magnésium, le stéarate de zinc, et leurs mélanges.

**16.** Composition selon l'une quelconque des revendications précédentes, **caractérisée en ce que** la phase grasse solide est présente en une teneur d'au moins 35 % en poids, notamment d'au moins 40 % en poids, et en particulier d'au moins 50 % en poids par rapport au poids total de la phase grasse.

**17.** Composition selon l'une quelconque des revendications précédentes, **caractérisée en ce que** la phase grasse solide est présente en une teneur allant de 10 à 22 % en poids, et en particulier de 10 à 20 % en poids par rapport au poids total de la composition.

**18.** Composition selon l'une quelconque des revendications précédentes, **caractérisée en ce que** le rapport pondéral phase grasse solide / charge(s) incompactable(s) ou charge(s) de faible(s) densité(s) de la composition selon l'invention varie de 1 à 4, notamment de 1 à 3, et en particulier de 1 à 2,5.

**19.** Composition selon l'une quelconque des revendications précédentes, **caractérisée en ce que** la phase grasse comprend en outre au moins une huile choisie parmi :

- l'huile de vison, l'huile de tortue, l'huile de soja, l'huile de pépins de raisin, l'huile de sésame, l'huile de maïs, l'huile de colza, l'huile de tournesol, l'huile de coton, l'huile d'avocat, l'huile d'olive, l'huile de ricin, l'huile de jojoba, l'huile d'arachide ;
- les huiles d'hydrocarbures ;
- les esters gras ;
- les huiles de silicone ;
- les acides gras supérieurs ;
- lés alcools gras supérieurs ;
- les poly méthylfluoroalkyl diméthylsiloxanes de formule (I) :

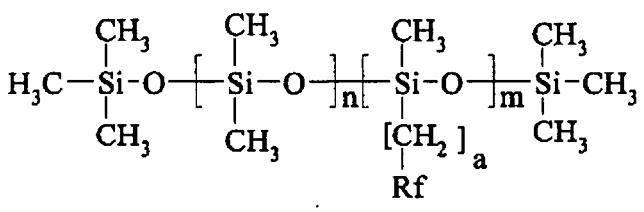

(I)

dans laquelle :

- n représente un entier variant de 5 à 90, notamment de 30 à 80 et en particulier de 50 à 80,
- m représente un entier variant de 1 à 150, notamment de 1 à 80, et en particulier de 1 à 40,
- a représente un entier variant de 0 à 5, et
- Rf désigne un radical perfluoroalkyle comprenant de 1 à 8 atomes de carbone ; et
- leurs mélanges.

**20.** Composition selon la revendication précédente, **caractérisée en ce que** le rapport pondéral phase grasse liquide / charge(s) incompactable(s) ou charge(s) de faible densité est inférieur ou égal à 3, notamment inférieur ou égal à 2,5, en particulier inférieur ou égal à 2, voire inférieur ou égal à 1,5.

**21.** Composition selon la revendication précédente, **caractérisée en ce que** la teneur en phase grasse liquide varie de 6 à 13 % en poids, et en particulier de 7 à 13 % en poids par rapport au poids total de la composition.

**22.** Composition selon l'une quelconque des revendications précédentes, **caractérisée en ce que** la teneur totale en phase grasse varie dé 15 à 30 % en poids par rapport au poids total de la composition.

**23.** Composition selon l'une quelconque des revendications précédentes, **caractérisée en ce qu'**elle comprend en outre au moins une charge additionnelle.

**24.** Composition selon la revendication précédente, **caractérisée en ce que** la charge additionnelle est choisie parmi le talc, le mica, la silice, le kaolin, les poudres de polyamide, de poly-β-alanine et de polyéthylène, les poudres de polymères de tétrafluoroéthylène, la lauroyl-lysine, l'amidon, le nitrure de bore, les poudres de polymères d'acide acrylique, les microbilles de résine de silicone, le carbonate de calcium précipité, le carbonate et l'hydrocarbonate de magnésium, l'hydroxyapatite, les microsphères de silice creuses, les microcapsules de verre ou de céramique.

**25.** Composition selon la revendication 26 ou 27, **caractérisée en ce que** la charge additionnelle est présente en une teneur variant de 40 à 95 % en poids, notamment de 45 à 85 % en poids, et en particulier de 45 à 70 % en poids, par rapport au poids total de la composition.

**26.** Composition selon l'une quelconque des revendications précédentes, **caractérisée en ce qu'**elle comprend en outre au moins une matière colorante pulvérulente.

**27.** Composition selon la revendication précédente, **caractérisée en ce que** la matière colorante pulvérulente est choisie parmi les pigments et les nacres.

**28.** Composition selon la revendication 26 ou 27, **caractérisée en ce que** la matière colorante pulvérulente est présente en une teneur variant de 0,5 à 30 % en poids, notamment de 1 à 22 % en poids, et particulier de 3 à 18 % en poids, par rapport au poids total de la composition.

**29.** Composition selon l'une quelconque des revendications précédentes, **caractérisée en ce qu'**elle comprend en outre, des agents colorants liposolubles ou hydrosolubles, des conservateurs, des actifs cosmétiques, des agents hydratants, des filtres UV, des épaississants, de l'eau, des tensioactifs et/ou des parfums.

**30.** Composition selon l'une quelconque des revendications précédentes, **caractérisée en ce qu'**elle se présente sous la forme d'un fard à joues, fard à paupières, d'une poudre pour le visage, d'un fond de teint, d'un produit anti-cernes, d'un produit de maquillage du corps ou d'un produit de soin pour le visage, d'un produit de soin du corps.

**31.** Composition selon la revendication précédente, **caractérisée en ce qu'**il s'agit d'un fond de teint.

**32.** Utilisation d'une phase pulvérulente telle que définie selon la revendication 1, d'une charge ou d'un mélange de charges incompactables et/ou de faible densité en une teneur supérieure à 5 % en poids par rapport au poids total de la composition en association avec une phase grasse comprenant au moins une phase grasse solide, pour la préparation d'une composition cosmétique de type poudre compacte dotée de propriétés de cohésion satisfaisantes.

**33.** Procédé de maquillage et/ou de soin de la peau comprenant au moins une étape d'application sur la peau d'au moins une composition selon l'une quelconque des revendications 1 à 31.

## Claims

**1.** Cosmetic composition in compact powder form for making up and/or caring for the skin, especially the face, comprising at least:

- a pulverulent phase,
- a solid fatty phase, and
- from 4% to 15% by weight relative to the total weight of the composition of a liquid fatty phase,

said pulverulent phase comprising more than 5% by weight relative to the total weight of the composition of at least an incompactable filler and/or low-density filler,
with the exclusion of a composition comprising 10% by weight of carnauba wax, 6% by weight of magnesium stearate and 5% by weight of amorphous magnesium and aluminium silicate relative to the total weight of the composition.

**2.** Cosmetic composition according to claim 1, wherein :

- the said solid fatty phase being present in an amount such that the compact powder has a cohesion corresponding to a loss of mass, evaluated by the drop test, of less than 12% by weight, and
- the said pulverulent phase being such that when it is used in a proportion of 91.2% by weight as a mixture

with 8.8% of a reference liquid binder described in Example 1 and when the whole is compacted at a pressure of $2 \times 10^7$ Pa, it results in a compact whose cohesion corresponds to a loss of mass, evaluated by the drop test, of greater than 12% by weight and/or a compact that does not erode homogeneously.

**3.** Composition according to any one of the preceding claims, **characterized in that** the said pulverulent phase comprises at least one pulverulent dyestuff.

**4.** Composition according to any one of the preceding claims, **characterized in that** the incompactable filler is chosen from incompactable fillers of mineral type, organic type, and mixtures thereof.

**5.** Composition according to any one of the preceding claims, **characterized in that** the incompactable filler is chosen from silica microspheres, microporous polymer microspheres, polyurethane powder, polymer microcapsules bearing only one closed cavity, and elastomeric crosslinked organopolysiloxane powders, and mixtures thereof.

**6.** Composition according to any one of the preceding claims, **characterized in that** the incompactable filler is chosen from silica microspheres of open porosity, hollow silica microspheres, microporous acrylic polymer microspheres, powdered copolymer of hexamethylene diisocyanate and of trimethylolhexyl lactone, microcapsules of methyl acrylate or methacrylate polymers or copolymers, or copolymers of vinylidene chloride and of acrylonitrile, and powders of elastomeric crosslinked organopolysiloxane coated with silsesquioxane resin.

**7.** Composition according to any one of the preceding claims, **characterized in that** the low-density filler has an untamped density ranging from 0.05 to 0.50 $g/cm^3$ and in particular ranging from 0.10 to 0.50 $g/cm^3$.

**8.** Composition according to the preceding claim, **characterized in that** the low-density filler has a tamped density ranging from 0.01 to 0.80 $g/cm^3$, especially ranging from 0.05 to 0.80 $g/cm^3$ and in particular ranging from 0.10 to 0.80 $g/cm^3$.

**9.** Composition according to any one of the preceding claims, **characterized in that** the said low-density filler is chosen from the fillers mentioned in Claims 6 to 8.

**10.** Composition according to any one of claims 4 to 12, **characterized in that** the said low-density filler or incompactable filler, or a mixture of the said fillers, is present in a content ranging from 5% to 15% by weight, especially from 5% to 13% by weight and in particular from 5% to 10% by weight relative to the total weight of the composition.

**11.** Composition according to any one of claims 1 to 13, **characterized in that** the solid fatty phase comprises at least one compound chosen from waxes and metal soaps, and mixtures thereof.

**12.** Composition according to any one of claims 1 to 14, **characterized in that** the solid fatty phase comprises at least one compound chosen from:

- beeswax, lanolin wax and Chinese insect waxes; rice wax, carnauba wax, candelilla wax, ouricury wax, cork fibre wax, sugarcane wax, Japan wax and sumach wax; montan wax; microcrystalline waxes, paraffin waxes, ozokerites, ceresin wax, lignite waxes, polyethylene waxes, the waxes obtained by Fisher-Tropsch synthesis, and fatty acid esters of glycerides that are solid at 40°C and especially above 55°C,
- the waxes obtained by catalytic hydrogenation of animal or plant oils containing linear or branched $C_8$-$C_{32}$ fatty chains, especially hydrogenated jojoba oil, hydrogenated sunflower oil, hydrogenated castor oil, hydrogenated coconut oil and hydrogenated lanolin oil,
- silicone waxes or fluoro waxes,
- zinc laurate, magnesium stearate, magnesium myristate and zinc stearate, and
- mixtures thereof.

**13.** Composition according to any one of the preceding claims, **characterized in that** the solid fatty phase comprises at least one compound chosen from paraffin waxes and carnauba waxes, and mixtures thereof.

**14.** Composition according to any one of the preceding claims, **characterized in that** the solid fatty phase comprises at least one wax that is at least partially in powder form, such as paraffin wax microbeads and carnauba wax microbeads.

**15.** Composition according to any one of the preceding claims, **characterized in that** the solid fatty phase comprises at least one metal soap chosen from zinc laurate, magnesium stearate, magnesium myristate and zinc stearate, and mixtures thereof.

**16.** Composition according to any one of the preceding claims, **characterized in that** the solid fatty phase is present in a content of at least 35% by weight, especially at least 40% by weight and in particular at least 50% by weight relative to the total weight of the fatty phase.

**17.** Composition according to any one of claims 1 to 19, **characterized in that** the solid fatty phase is present in a content ranging from 10% to 22% by weight and in particular from 10% to 20% by weight relative to the total weight of the composition.

**18.** Composition according to any one of claims 2 to 20, **characterized in that** the solid fatty phase/incompactable filler (s) or low-density filler(s) weight ratio of the composition according to the invention ranges from 1 to 4, especially from 1 to 3 and in particular from 1 to 2.5.

**19.** Composition according to any one of the preceding claims, **characterized in that** the fatty phase also comprises at least one oil chosen from:

- mink oil, turtle oil, soybean oil, grapeseed oil, sesame seed oil, corn oil, rapeseed oil, sunflower oil, cotton seed oil, avocado oil, olive oil, castor oil, jojoba oil and groundnut oil;
- hydrocarbon oils;
- fatty esters;
- silicone oils;
- higher fatty acids;
- higher fatty alcohols;
- polymethylfluoroalkyldimethylsiloxanes of formula (I):

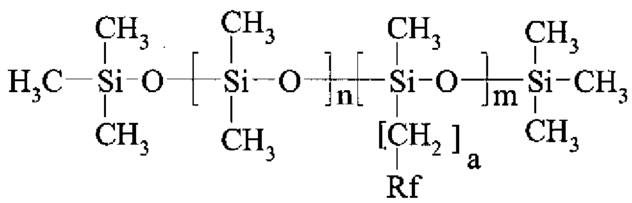

(I)

in which:

- n represents an integer ranging from 5 to 90, especially from 30 to 80 and in particular from 50 to 80,
- m represents an integer ranging from 1 to 150, especially from 1 to 80 and in particular from 1 to 40;
- a represents an integer ranging from 0 to 5, and
- Rf denotes a perfluoroalkyl radical containing from 1 to 8 carbon atoms; and
- mixtures thereof.

**20.** Composition according to the preceding claim, **characterized in that** the liquid fatty phase/incompactable filler(s) or low-density filler(s) weight ratio is less than or equal to 3, especially less than or equal to 2.5 and in particular less than or equal to 2, or even less than or equal to 1.5.

**21.** Composition according to the preceding claim, **characterized in that** the content of liquid fatty phase ranges from 6% to 13% by weight and in particular from 7% to 13% by weight relative to the total weight of the composition.

**22.** Composition according to any one of the preceding claims, **characterized in that** the total content of fatty phase ranges from 15% to 30% by weight relative to the total weight of the composition.

**23.** Composition according to any one of the preceding claims, **characterized in that** it also comprises at least one additional filler.

**24.** Composition according to the preceding claim, **characterized in that** the additional filler is chosen from talc, mica, silica, kaolin, polyamide powder, poly-β-alanine powder and polyethylene powder, tetrafluoroethylene polymer powders, lauroyllysine, starch, boron nitride, acrylic acid polymer powders, silicone resin microbeads, precipitated calcium carbonate, magnesium carbonate, magnesium hydrocarbonate, hydroxyapatite, hollow silica microspheres and glass or ceramic microcapsules.

**25.** Composition according to claim 26 or 27, **characterized in that** the additional filler is present in a content ranging from 40% to 95% by weight, especially from 45% to 85% by weight and in particular from 45% to 70% by weight relative to the total weight of the composition.

**26.** Composition according to any one of the preceding claims, **characterized in that** it also comprises at least one pulverulent dyestuff.

**27.** Composition according to the preceding claim, **characterized in that** the pulverulent dyestuff is chosen from pigments and nacres.

**28.** Composition according to claim 26 or 27, **characterized in that** the pulverulent dyestuff is present in a content ranging from 0.5% to 30% by weight, especially from 1% to 22% by weight and in particular from 3% to 18% by weight relative to the total weight of the composition.

**29.** Composition according to any one of the preceding claims, **characterized in that** it also comprises liposoluble or water-soluble colouring agents, preserving agents, cosmetic active agents, moisturizers, UV-screening agents, thickeners, water, surfactants and/or fragrances.

**30.** Composition according to any one of the preceding claims, **characterized in that** it is in the form of a makeup rouge, an eyeshadow, a face powder, a foundation, a concealer product, a body makeup product, a facial care product or a body care product.

**31.** Composition according to the preceding claim, **characterized in that** it is a foundation.

**32.** Use of a pulverulent phase as defined according to claim 1, of an incompactable filler or a mixture of incompactable fillers and/or low-density fillers in a content of greater than or equal to 5% by weight relative to the total weight of the composition, in combination with a fatty phase comprising at least one solid fatty phase, for the preparation of a cosmetic composition of compact powder type that has satisfactory cohesion properties.

**33.** Process for making up and/or caring for the skin, comprising at least one step of applying to the skin at least one composition according to any one of claims 1 to 31.

**Patentansprüche**

**1.** Kosmetische Zusammensetzung in Form von Kompaktpuder zum Schminken und/oder zum Pflege der Haut, insbesondere des Gesichts, die mindestens folgendes umfasst:

- eine pulverförmige Phase,
- eine feste Fettphase, und
- 4 bis 15 Gew.-%, bezogen auf das Gesamtgewicht der Zusammensetzung, einer flüssigen Fettphase,

wobei die pulverförmige Phase mehr als 5 Gew.-% bezogen auf das Gesamtgewicht der Zusammensetzung von mindestens einem nicht kompaktierbaren und/oder niedrigdichten Füllstoff umfaßt,
ausgenommen eine Zusammensetzung, die 10 Gew.-% Carnaubawachs, 6 Gew.-% Magnesiumstearat und 5 Gew.-% amorphes Magnesium- und Aluminiumsilicat bezogen auf das Gesamtgewicht der Zusammensetzung umfasst.

**2.** Zusammensetzung nach Anspruch 1, wobei

- die feste Fettphase in einer solchen Menge vorhanden ist, dass der Kompaktpuder eine Kohäsion aufweist, die einem mit dem Falltest bestimmten Masseverlust von weniger als 12 Gew.-% entspricht, und
- die pulverförmige Phase so ist, dass wenn sie in einem Verhältnis von 91,2 Gew.% im Gemisch mit 8,8 % von einem flüssigen Referenzbindemittel verwendet wird, nämlich eine Zusammensetzung, enthaltend 40,91 g Ethyl-2-hexyl-para-methoxy-4-cinnamat. 25 g Isocetylstearat, 25 g Polydimethylsiloxan 10 cst und 9.09 g eines Konservierungsmittelgemisches, enthaltend 70 % Phenoxy-2-ethanol, 17.4 % Methyl-p-hydroxybenzoat, 4.2 % Ethyl-p-hydroxybenzoat, 4.2 % Butyl-p-hydroxybenzoat, 2,1% Propyl-p-hydroxybenzoat und 2.1 % Isobutyl-p-hydroxybenzoat, und das ganze mit einem Druck von $2x10^7$ Pa kompaktiert wird, sich ein Preßling, dessen Kohäsion einem Falltest-Masseverlust von größer als 12 Gew.-% entspricht, und/oder ein Preßling, der nicht homogen zerfällt, ergibt.

3. Zusammensetzung nach einem der vorhergehenden Ansprüche, **dadurch gekennzeichnet, dass** die pulverförmige Phase weiterhin mindestens einen pulverförmigen Farbstoff umfasst.

4. Zusammensetzung nach einem der vorhergehenden Ansprüche, **dadurch gekennzeichnet, dass** der nicht kompaktierbare Füllstoff aus nicht kompaktierbaren mineralischen Füllstoffen, organischen Füllstoffen und ihren Gemischen ausgewählt ist.

5. Zusammensetzung nach einem der vorhergehenden Ansprüche, **dadurch gekennzeichnet, dass** der nicht kompaktierbare Füllstoff ausgewählt ist aus Siliciumdioxid-Mikrokegeln, mikroporösen Mikrokugeln von Polymeren, Polyurethanpulver, Mikrokapseln von Polymeren, die einen einzigen abgeschlossenen Hohlraum umfassen, Pulvern von vernetzten elastomeren Polyorganosiloxanen und ihren Gemischen.

6. Zusammensetzung nach einem der vorhergehenden Ansprüche, **dadurch gekennzeichnet, dass** der nicht kompaktierbare Füllstoff ausgewählt ist aus Siliciumdioxid-Mikrokugeln mit offener Porosität, Siliciumdioxid-Mikrohohlkugeln, mikroporösen Mikrokugeln von Acrylpolymeren, Copolymer-Pulver von Hexamethylendiisocyanat und Trimethylolhexyllacton, Mikrokapseln von Acrylat-, Methylmethacrylat-Polymeren oder Copolymeren oder von Vinylidenchlorid- und Acrylnitril-Copolymeren, Pulvern von mit Silsesquioxan umhülltem vernetztem elastomerem Polyorganosiloxan.

7. Zusammensetzung nach einem der vorhergehenden Ansprüche, **dadurch gekennzeichnet, dass** der niedrigdichte Füllstoff eine nicht komprimierte Dichte von 0,05 bis 0,50 $g/cm^3$ und insbesondere von 0,10 bis 0,50 $g/cm^3$ aufweist.

8. Zusammensetzung nach einem der vorhergehenden Ansprüche, **dadurch gekennzeichnet, dass** der niedrigdichte Füllstoff eine komprimierte Dichte von 0,01 bis 0.80 $g/cm^3$, insbesondere von 0,05 bis 0.80 $g/cm^3$ und speziell von 0,10 bis 0,80 $g/cm^3$ aufweist.

9. Zusammensetzung nach einem der vorhergehenden Ansprüche, **dadurch gekennzeichnet, dass** der niedrigdichte Füllstoff aus den in Anspruch 6 bis 8 aufgeführten Füllstoffen ausgewählt ist.

10. Zusammensetzung nach einem der vorhergehenden Ansprüche, **dadurch gekennzeichnet, dass** der niedrigdichte oder nicht kompaktierbare Füllstoff oder ein Gemisch der Füllstoffe in einem Gehalt von 5 bis 15 Gew.-% insbesondere von 5 bis 13 Gew.-% und speziell von 5 bis 10 Gew.-% bezogen auf das Gesamtgewicht der Zusammensetzung vorhanden ist.

11. Zusammensetzung nach einem der vorhergehenden Ansprüche, **dadurch gekennzeichnet, dass** die feste Fettphase mindestens eine Verbindung umfasst, die aus Wachsen, Metallseifen und ihren Gemischen ausgewählt ist.

12. Zusammensetzung nach einem der vorhergehenden Ansprüche, **dadurch gekennzeichnet, dass** die feste Festphase mindestens eine Verbindung umfasst, die ausgewählt ist aus:

- Bienenwachs, Lanolinwachs, Chinainsektenwachsen, Reiswachs, Carnaubawachs, Candelillawachs, Ouricouriwachs, Wachs von Korkfasern, Zuckerrohrwachs, Japanwachs und Sumachwachs: Montanwachs, mikrokristallinenen Wachsen, Paraffinwachsen, Ozokeriten, Ceresinwachs, Lignitwachs, Polyethylenwachsen, durch Fischer-Tropsch-Synthese erhaltenen Wachsen, Estern von Fettsäuren und aus Glyzeriden, die bei 40 °C und insbesondere bei mehr als 50°C fest werden,
- durch katalytische Hydrierung von tierischen oder pflanzlichen Ölen mit linearen oder verzweigten $C_3$-$C_{32}$-Fettsäureketten erhaltenen Wachsen, insbesondere hydriertes Jojobaöl, hydriertes Sonnenblumenöl, hydriertes

Rizinusöl, hydriertes Coprahöl und hydriertes Lanolinöl,
- Wachsen von Silikonen oder fluorierten Wachsen,
- Zinklaureat; Magnesiumstearat; Magnesiummyristat, Zinkstearat, und
- ihren Gemischen.

**13.** Zusammensetzung nach einem der vorhergehenden Ansprüche, **dadurch gekennzeichnet, dass** die feste Fettphase mindestens eine Verbindung umfasst, die aus Paraffin-wachsen. Carnaubawachsen und ihren Gemischen ausgewählt ist.

**14.** Zusammensetzung nach einem der vorhergehenden Ansprüche, **dadurch gekennzeichnet, dass** die feste Fettphase mindestens ein Wachs mindestens zum Teil in Pulverform, wie Paraffinwachs-Mikrokugeln und Carnaubawachs-Mikrokugeln, umfaßt.

**15.** Zusammensetzung nach einem der vorhergehenden Ansprüche, **dadurch gekennzeichnet, dass** die feste Fettphase mindestens eine Metallseife umfasst, die aus Zinklaureat, Magnesiumstearat, Magnesiummyristat, Zinkstearat und ihren Gemischen ausgewählt ist.

**16.** Zusammensetzung nach einem der vorhergehenden Ansprüche, **dadurch gekennzeichnet, dass** die feste Fettphase in einem Gehalt von mindestens 35 Gew.-%, insbesondere von mindestens 40 Gew.-% und speziell von mindestens 50 Gew.-% bezogen auf das Gesamtgewicht der Fettphase vorhanden ist.

**17.** Zusammensetzung nach einem der vorhergehenden Ansprüche, **dadurch gekennzeichnet, dass** die feste Fettphase in einem Gehalt von 10 bis 22 Gew.-% und insbesondere von 10 bis 20 Gew.-% bezogen auf das Gesamtgewicht der Zusammensetzung vorhanden ist.

**18.** Zusammensetzung nach einem der vorhergehenden Ansprüche, **dadurch gekennzeichnet, dass** das Gewichtverhältnis feste Fettphase/nicht kompaktierbare(r) Füllstoff(e) oder niedrigdichte(r) Füllstoff(e) der erfindungsgemäßen Zusammensetzung von 1 bis 4, insbesondere von 1 bis 3 und speziell von 1 bis 2,5 variiert.

**19.** Zusammensetzung nach einem der vorhergehenden Ansprüche, **dadurch gekennzeichnet, dass** die Fettphase weiterhin mindestens ein Öl umfasst, das ausgewählt ist aus:

- Nerzöl, Schildkrötenöl, Sojaöl, Traubenkernöl, Sesamöl, Maisöl, Colzaöl, Sonnenblumenöl, Baumwollöl, Avocadoöl, Olivenöl, Rizinusöl, Jojobaöl, Erdnussöl;
- Kohlenwasserstoffölen;
- Fettsäurestern;
- Siliconölen;
- höheren Fettsäure:
- höheren Fettalkoholen:
- Polymethylfluoralkyldimethylsiloxanen der Formel (I):

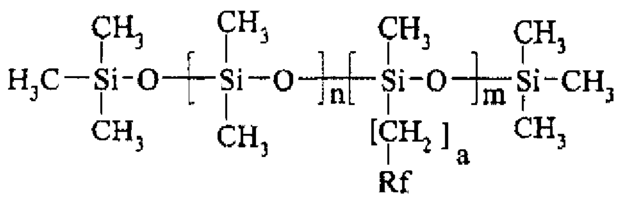

(I)

wobei:

- n eine ganze Zahl darstellt, die von 5 bis 90, insbesondere von 30 bis 80 und speziell von 50 bis 80 variiert,
- m eine ganze Zahl darstellt, die von 1 bis 150, insbesondere von 1 bis 80 und speziell von 1 bis 40 variiert,
- a eine ganze Zahl darstellt, die von 0 bis 5 variiert, und
- Rf einen Perfluoralkylrest bezeichnet, der 1 bis 8 Kohlenstoffatome einschließt; und

- ihren Gemischen.

**20.** Zusammensetzung nach dem vorhergehenden Anspruch, **dadurch gekennzeichnet, dass** das Gewichtsverhältnis flüssige Fettphase/nicht kompaktierbare(r) Füllstoff(e) oder niedrigdichte(r) Füllstoff(e) kleiner oder gleich 3, insbesondere kleiner oder gleich 2,5, speziell kleiner oder gleich 2 und sogar kleiner oder gleich 1,5 ist.

**21.** Zusammensetzung nach einem der vorhergehenden Ansprüche, **dadurch gekennzeichnet**, das der Gehalt an flüssiger Fettphase von 6 bis 13 Gew.-% und insbesondere von 7 bis 13 Gew.-% bezogen auf das Gesamtgewicht der Zusammensetzung variiert.

**22.** Zusammensetzung nach einem der vorhergehenden Ansprüche, **dadurch gekennzeichnet, dass** der Gesamtgehalt an Fettphase von 15 bis 30 Gew.-% bezogen auf das Gesamtgewicht der Zusammensetzung variiert.

**23.** Zusammensetzung nach einem der vorhergehenden Ansprüche, **dadurch gekennzeichnet, dass** sie weiterhin mindestens einen zusätzlichen Füllstoff umfasst.

**24.** Zusammensetzung nach dem vorhergehenden Ansprüche, **dadurch gekennzeichnet**, der zusätzliche Füllstoff ausgewählt ist aus Talk, Mica, Siliciumdioxid, Kaolin, Pulvern von Polyamid. Poly-β-alanin und Polyethylen, Pulvern von Tetrafluorethylenpolymeren, Lauroyllysin, Stärke, Bornitrid, Pulvern von Acrylsäurepolymeren, Siliconharz-Mikrokugeln, Calciumcarbonat-Präzipitat, Magnesiumcarbonat und Magnesiumhydrogencarbonat, Hydroxylapatit, Silciumdioxid-Mikrohohlkugeln, Glas- oder Keramik-Mikrokapseln.

**25.** Zusammensetzung nach Anspruch 26 oder 27, **dadurch gekennzeichnet, dass** der zusätzliche Füllstoff in einem Gehalt vorhanden ist, der von 40 bis 95 Gew.-%, insbesondere von 45 bis 85 Gew.-% und speziell von 45 bis 70 Gew.-% bezogen auf das Gesamtgewicht der Zusammensetzung variiert.

**26.** Zusammensetzung nach einem der vorhergehenden Ansprüche, **dadurch gekennzeichnet, dass** sie weiterhin mindestens einen pulverförmigen Farbstoff umfasst.

**27.** Zusammensetzung nach dem vorhergehenden Ansprüche, **dadurch gekennzeichnet, dass** der pulverförmige Farbstoff aus Pigmenten und Perlmuttlacken ausgewählt ist.

**28.** Zusammensetzung nach Anspruch 26 oder 27, **dadurch gekennzeichnet**, der pulverförmige Farbstoff in einem Gehalt vorhanden ist, der von 0,5 bis 30 Gew.-%, insbesondere von 1 bis 22 Gew.-% und speziell von 3 bis 18 Gew.-% bezogen auf das Gesamtgewicht der Zusammensetzung variiert.

**29.** Zusammensetzung nach einem der vorhergehenden Ansprüche, **dadurch gekennzeichnet, dass** sie weiterhin fett- oder wasserlösliche Farbmittel, Konservierungsstoffe, kosmetische Wirkstoffe, hydratisierende Mittel. UV-Filter, Verdickungsmittel, Wasser, grenzflächenaktive Mittel und/oder Duftstoffe umfasst.

**30.** Zusammensetzung nach einem der vorhergehenden Ansprüche, **dadurch gekennzeichnet, dass** sie in der Form von einem Wangenrouge, Lidschatten, Gesichtspuder. Teintgrundierung. Antifaltenprodukt, Körperschminkprodukt oder einem Geschichtspflegeprodukt. Körperpflegeprodukt vorliegt.

**31.** Zusammensetzung nach dem vorhergehenden Ansprüche, **dadurch gekennzeichnet, dass** es sich um eine Teintgrundierung handelt.

**32.** Verwendung einer pulverförmigen Phase, wie in Anspruch 1 definiert, eines Füllstoffs oder eines Gemisches von nicht kompaktierbaren und/oder niedrigdichten Füllstoffen in einem Gehalt von größer als 5 Gew.-% bezogen auf das Gesamtgewicht der Zusammensetzung in Kombination mit einer Fettphase, die mindestens eine feste Fettphase umfasst, zur Herstellung einer kosmetischen Zusammensetzung vom Typ eines Kompaktpuders, der mit zufriedenstellenden Kohäsionseigenschaften ausgestattet ist.

**33.** Verfahren zum Schminken und/oder Pflegen der Haut, umfassend mindestens einen Schritt des Ausbringens auf die Haut von mindestens einer Zusammensetzung nach einem der Ansprüche 1 bis 31.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9317660 A
- EP 0717979 A
- EP 1277458 A
- EP 0132631 A
- FR 2798287
- EP 1159950 A
- EP 0657486 A
- EP 1086683 A
- US 3615972 A
- EP 056219 A
- JP 2243612 A
- US 5538793 A